Europäisches Patentamt

European Patent Office

Office européen des brevets

(11) Publication number: **0 467 466 A1**

# EUROPEAN PATENT APPLICATION

(21) Application number: **91201830.6**

(22) Date of filing: **12.07.91**

(51) Int. Cl.5: **G01N 33/535**, G01N 33/78, G01N 33/546, G01N 33/548, G01N 33/58

(30) Priority: **16.07.90 US 552960**
**10.07.91 US 727769**

(43) Date of publication of application:
**22.01.92 Bulletin 92/04**

(84) Designated Contracting States:
**AT BE CH DE DK ES FR GB GR IT LI LU NL SE**

(71) Applicant: **EASTMAN KODAK COMPANY**
**343 State Street**
**Rochester, New York 14650-2201(US)**

(72) Inventor: **Hilborn, David Alan, c/o EASTMAN KODAK COMPANY**
**Patent Department, 343 State Street**
**Rochester, New York 14650-2201(US)**
Inventor: **Danielson, Susan Jean, c/o**
**EASTMAN KODAK COMPANY**
**Patent Department, 343 State Street**
**Rochester, New York 14650-2201(US)**

(74) Representative: **Nunney, Ronald Frederick Adolphe et al**
**Kodak Limited Patent Department Headstone Drive**
**Harrow Middlesex HA1 4TY(GB)**

(54) Method for the purification of immunoreactive labeled thyroxine conjugates.

(57) A method for purifying immunoreactive labeled thyroxine conjugates using immobilied antibodies that are i) raised against an analogue of thyroxine (T4) and ii) cross-reactive with thyroxine.

ELUTION PROFILE OF T4-ALP CONJUGATE ON A T3 AGROSE AFFINITY COLUMN

FIG.2

The present invention relates to immunoassays and in particular to a method of improving the immunoreactivity of labeled thyroxine conjugates used in immunoassays.

Thyroxine (T4) is an important hormone in the mammalian physiology, being excreted by the thyroid gland. The measurement of thyroxine is an important diagnostic tool in the determination of disease. Various techniques have been used for the determination of thyroxine, including radioimmunoassay, competitive protein binding, chromatography, and so forth

Conjugates of T4 (thyroxine) and enzymes have been employed in immunoassays for T4. In most of these assays, the T4-enzyme conjugate and the T4 in the sample compete for T4 antibody binding sites on T4 antibodies that have been immobilized on a solid surface in a competitive, heterogeneous assay. Such surfaces include the walls of test tubes, the wells of microtiter plates and the surface of polymeric beads.

When the concentration of T4 in the sample is high compared to the concentration of T4-enzyme employed in the assays, relatively more T4 and less T4-enzyme conjugate bind to the immobilized antibody so that there is an inverse correlation between the amount of T4-enzyme bound to the immobilized antibody and the amount of T4 in the sample. In these assays, the immobilizing surface is washed extensively after the binding reaction has occurred to remove any free (unbound) T4-enzyme. Then a solution containing the substrate of the enzyme is added so it is in contact with the immobilized enzyme. The substrate is chosen so that an observable change occurs (for example, a change in color) as the enzyme acts on the substrate. This change is a measure of the amount of enzyme conjugate that is bound to the immobilized antibody and is thus related to the amount of T4 in the original sample.

The problem is that the utility of T4-enzyme conjugates is compromised if some of the enzyme in the T4-enzyme preparation is incapable of being bound by the immobilized antibody (non-immunoreactive T4-enzyme conjugate). This could happen if some of the enzyme has not been derivatized with T4 or if the derivatized T4 is not accessible to the T4 antibody binding site. Non-immunoreactive T4-enzyme contributes enzyme activity without contributing to the competition reaction. This may result in either lower final signal or higher background (non-specific) signal. If high background signal is the problem, this excess signal must be removed from the final measurement, usually by extensively washing the immobilized antibodies.

Figure 1 is a graph showing the immunoreactivity of unpurified labeled T4 conjugates.

Figure 2 is a graph showing alkaline phosphatase activity of the fractions eluted from the affinity columns.

Figures 3A and 3B are graphs showing the immunoreactivity of the eluants of Figure 2.

Figure 4 is a graph showing assay results for T4 using a labeled T4 conjugate of the example.

The present invention provides a method for purifying immunoreactive labeled thyroxine conjugates that obviates many of the problems of the prior art. The method comprises the steps of:

(a) immobilizing antibodies that have an affinity for thyroxide (T4);

(b) preparing a crude liquid mixture of labeled thyroxine conjugate;

(c) passing the liquid mixture of labeled thyroxine conjugate over the immobilized antibodies;

(d) washing the immobilized antibodies with a buffer solution having a pH of 4 to 9; and

(e) passing a buffered solution of thyroxine over the immobilized antibodies, thereby removing immunoreactive labeled thyroxine conjugates characterized in that the immobilized antibodies are i) raised against an analogue of thyroxine (T4) and ii) cross-reactive with thyroxine.

The method of this invention provides labeled thyroxine conjugates that are greater than 75% immunoreactive preferably 75 to 100 % immunoreactive.

The novel feature in the purification method described herein is the use of a non-T4 affinity column to purify a T4-enzyme conjugate. The reduced affinity between T4 and antibodies that are i) raised against an analogue of thyroxine (T4) and ii) cross-reactive with thyroxine affords easier removal of the affinity column retained material than with T4 affinity columns.

This invention provides a method of obtaining a labeled T4 conjugate, particularly a T4-enzyme preparation containing a much higher level of immunoreactivity than available heretofore. The method will be demonstrated specifically with the enzyme alkaline phosphatase (ALP) but it will be understood by those skilled in the art that the principles involved apply to any label. It will also be clear that the products of the method are useful in any immunoassay for T4. The method used to purify the immunoreactive labeled T4 conjugate involves removing the non-immunoreactive components by affinity chromatography. It will be understood by those skilled in the art that affinity chromatography can be carried out with or without a column. For example simply mixing the immobilized antibodies with a sample of the labeled T4 conjugate and completing the other steps of the method as described would also be successful.

Affinity chromatography employs an immobilized antibody on a solid support that recognizes a subset of the components of a solution and binds that subset of components so that one subset of components can be separated from the other. However there may be a strong interaction between the antibody used on the solid support in the affinity chromatography step and the desired component of the solution being purified. When this occurs, the desired component is retained by the immobilized antibody, separating it from undesirable components. But it is difficult to remove the desired component from the immobilized antibody to make it available for use. This occurs when a T4 antibody is immobilized for use in the purification of a labeled T4 conjugate.

In this invention, this difficulty is obviated by using an antibody that binds T4 weakly compared to a usual T4 antibody so that the removal of the desired component, in this case, immunoreactive labeled T4 conjugates, from the immobilized antibody is simplified. The antibody used in the affinity purification step is one that is a raised against a T4 analogue and has a cross-reactivity with T4. Examples of T4 analogues include 3,3',5-triiodothyronine (T3); 3,5-diiodothyronine (T2); or 3,3',5'-triiodothyronine all of which have a weak reactivity towards T4. Thus, these antibodies have the desired characteristics of recognizing T4 so that it can retain immunoreactive T4-ALP with a weak binding interaction. This weak binding also allows the immunoreactive labeled T4 conjugate to be easily removed.

The term "T4 analogue" is intended in this application to refer to compounds which are structurally and chemically similar, but not identical, to T4. For example, the term is not intended to mean the same as the term "ligand analogue" which has a well recognised meaning in immunology. One such immunological use of the term "ligand analogue" is found in EP-B-0 103 605 (published March 28, 1984) and in the publications mentioned therein, such as EP-A-0 026 103 (published April 1, 1981)

There are a variety of solid supports for immobilizing the antibodies such as agarose (a purified form of polysaccharide agar), cellulose, dextran and glass beads. Another particularly useful support for the antibodies, especially for high performance affinity chromatographic methods, is a polymer particle of addition-polymerizable vinyl (including acrylic) monomers.

The polymeric particles are generally water-insoluble latex particles having an average particle size greater than 0.01 micrometers. Preferably they have an average particle size in the range of from 0.01 to 10, preferably 0.3 to 3 micrometers.

Preferred polymers can be represented by the formula (III):

$$( A )x ( B )y ( D )z \quad (III)$$

wherein -A- represents recurring units derived from one or more hydrophobic ethylenically unsaturated monomers,

-B- represents recurring units derived from one or more ethylenically unsaturated monomers having the requisite reactive groups through which the polymer particle can be appended to form ligand analogues;

-D- represents recurring units derived from one or more ethylenically unsaturated monomers which are different than those represented by -A- or -B-.

In formula (III), x is from 0 to 99.9 mole percent, y is from 0.1 to 100 mole percent and z is from 0 to 20 mole percent. Preferably, x is from 45 to 99.5 mole percent, y is from 0.5 to 50 mole percent, and z is from 0 to 10 mole percent. Polymer beads according to formula III are disclosed in EPA 0 308 233A; 0 323 692 A; 0 280 556A; and 0 302 715A and Kokai 89/0054258 and 89/0054259, each of which are expressly incorporated herein by reference.

Monomers from which the -A- recurring units are selected are hydrophobic and form homopolymers that are insoluble in water. Preferably, these monomers have aromatic groups. Representative hydrophobic monomers include, but are not limited to, styrene and styrene derivatives (for example, 4-vinyltoluene, 2,5-di-methylstyrene, 4-t-butylstyrene, 2-chlorostyrene and others known in the art), acrylic and methacrylic acid esters and amides (for example, n-butyl acrylate, propyl methacrylate, methyl acrylate, methyl methacrylate, ethyl methacrylate, 2-ethylhexyl methacrylate, N-phenylacrylamide and others known in the art), acrylonitrile and vinyl acetate.

The monomers from which the -B- recurring units are selected are vinylbenzyl chloride, vinylbenzyl bromide, m- and p-(2-chloroethylsulfonylmethyl)styrene, N-(4-chloroethylsulfonylmethylphenyl)acrylamide, vinyl chloroacetate, N-(3-chloroacetamidopropyl)methacrylamide, 2-chloroacetamidoethyl methacrylate, 4-chloroacetamidostyrene, m- and p-chloroacetamidomethylstyrene, N-(3-chloroacetamidocarbonyliminopropyl)-methacrylamide, 2-chloroacetamidocarbonyliminoethyl methacrylate, 4-chloroacetamidocarbonyliminostyrene, m- and p-chloroacetamidocarbonyliminomethylstyrene, N-vinyl-N'-(3-chloropropionyl)urea, 4,(3-chloropropionamido)styrene, 4-(3-chloropropionamidocarbonylimino)styrene, 2-(3-chloropropionamido)ethyl methacrylate, N-[2-(3-chloropropionamido)ethyl]methacrylamide, acrylic acid, methacrylic acid, glycidyl acrylate, glycidyl methacrylate, vinylbenzaldehyde and N-(3-aminopropyl)methacrylamide hydrochloride.

Monomers from which the -D- recurring units are derived include monomers different than those from which -A- and -B- are derived. Specifically, the -D-recurring units are derived from monomers which impart aqueous dispersion stability to the particles or other properties. Representative monomers include, but are not limited to, anionic monomers such as sodium 2-acrylamido-2-methylpropanesulfonate, acrylic acid, methacrylic acid, 2-carboxyethyl acrylate, styrene sulfonic acid, potassium salt and m & p-carboxymethylstyrene and other ethylenically unsaturated polymerizable sulfonates, carboxylates, sulfates and phosphonates, other hydrophilic but nonionic monomers, such as 2-hydroxyethyl acrylate and 2-hydroxyethyl methacrylate and others known to one skilled in the art.

Preferred monomers from which the -D- units are derived are acrylic acid, methacrylic acid, sodium 2-acrylamido-2-methylpropanesulfonate, m & p-carboxymethylstyrene and p-styrenesulfonic acid, potassium salt.

Representative polymers of the monomers described above include the following: poly(m & p-chloromethylstyrene), poly(styrene-co-m & p-chloromethylstyrene-co-2-hydroxyethyl acrylate) (67:30:3 molar ratio), poly(styrene-co-m & p-chloroethylsulfonylmethylstyrene) (95.5:4.5 molar ratio), poly ¢styrene-co-N-[m & p-(2-chloroethylsulfonylmethyl)phenyl]acrylamide/ (99.3:0.7 molar ratio), poly(m & p-chloromethylstyrene-co-methacrylic acid) (95:5, 98:2 and 99.8:0.2 molar ratio), poly(styrene-co-m & p-chloroethylsulfonylmethylstyrene-co-methacrylic acid) (93.5:4.5:2 molar ratio), poly ¢styrene-co-N-[m & p- (2-chloroethylsulfonylmethyl)phenyl]-acrylamide-co-methacrylic acid/(97.3:0.7:2 molar ratio), and poly(styrene- co-m & p-chloromethylstyrene)(70:30 molar ratio).

The antibody can be attached directly to the support or alternatively either the antibody or the support can be derivatized prior to attachment. Spacers arms are commonly attached to either the antibody or the support to provide greater accessibility of the attached antibody.

Coupling techniques commonly used for the attachment of proteins to supports involve the reaction of nucleophilic groups on the protein such as amino, sulfhydryl and hydroxyl groups, with a variety of electrophilic groups on the carrier.

Amino groups of proteins are commonly reacted with epoxides, aldehydes, isothiocyanate groups, vinyl groups or active esters on the support. Sulfhydryl groups of the proteins can form thioester or disulfide bonds. Hydroxyl groups which are found on agarose or silica based supports are commonly activated prior to reaction with cyanogen bromide, chloroformates, 1,1'-carbonyldiimidazole, sulfonyl chlorides and tresylchloride. Nucleophilic groups on the supports have also been reacted with electrophilic groups on the protein. The carbohydrate residues of certain proteins including antibodies can be oxidized to aldehydes which will react with amine or hydrazide groups on the support.

The purified labeled T4 conjugates are useful in any immunoassay for T4. The assay may be competitive as described supra, homogenous or heterogeneous as those terms are understood in the art. See, for example U.S. Patent 4,670,381. The assay may be direct or indirect. ELISA, EMIT and FIA are well known examples of such immunoassays.

In a broad aspect the present invention is applicable to an immunoassay for T4, comprising the steps of:

(a) providing a labeled T4 conjugate according to the invention,
(b) mixing a sample containing T4 with (a);
(c) reacting the mixture from (b) with a known amount of an antibody for T4, and
(d) measuring the amount of label either bound or unbound to the T4 antibody.

The labeled T4 conjugates are useful in dry analytical elements designed to carry out immunoassays. Such elements typically comprise a support layer, a reagent zone, and a spreading zone. The two zones can be combined into a single layer or can be separate layers. The element can comprise one or more layers, for example separate or combined reagent/spreading layer and a gelatin buffer layer containing other necessary additives, coupling enzymes, and so forth The beads can include both large and small polymeric beads, and they can either be coated in the same or different layers. The small beads can be coated before, concurrently with, or after the large beads.

The reagent layer or the spreading layer of the element can contain the indicator composition comprising one or more reagents dispersed in one or more synthetic or natural binder materials, such as gelatin, or other naturally-occurring colloids, homopolymers and copolymers, such as poly-(acrylamide), poly(vinyl pyrrolidone), poly(N-isopropylacrylamide), poly(acrylamide-co-N-vinyl-2-pyrrolidone) and similar copolymers.

Other optional layers, for example subbing layers, radiation-blocking layers, and so forth can be included if desired. All layers of the element are in fluid contact with each other, meaning that fluids and reagents and uncomplexed reaction products in the fluids can pass between superposed regions of adjacent layers.

The various immunoassays provided by this invention assay can be carried out using any suitable label which can be attached to T4. Useful labels may include radioactive tags, dyes, fluorescers, enzymes, enzyme substrates, enzyme inhibitors, allosteric effectors, cofactors and other known enzyme modulators. Enzymes, such as glucose oxidase, peroxidase, alkaline phosphatase (ALP), horseradish peroxidase (HRP), including amine-enriched horseradish peroxidase, and galactosidase are preferred labels.

When an enzyme label is used, the substrate for the enzyme is present in the element or added thereto in the wash liquid. The substrate can be added to the element prior to or simultaneously with the liquid sample, or after completion of the binding reaction. It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. For example, if the enzyme label is peroxidase, the substrate is hydrogen peroxide. Using glucose oxidase as an example, the substrate glucose is generally present in the reagent layer or added in the wash liquid to yield 0.01 moles/m$^2$, and preferably from 0.001 to 0.1 mole/m$^2$. A worker skilled in the art would know how to adjust the amount of a particular substrate for the amount of enzyme label used in the assay.

When certain labels are used, for example enzymes, cofactors, enzyme substrates or enzyme modulators, the reagent layer contains an indicator composition comprising one or more reagents which provide a detectable species as a result of reaction of the label. Preferably, the indicator composition is a colorimetric indicator composition which provides a colorimetrically detectable species as a result of enzymatic reaction of an enzyme-labeled ligand analogue with a substrate.

The indicator composition can be a single compound which produces a detectable dye upon enzymatic reaction, or a combination of reagents which produce the dye. For example, when glucose is used as the substrate and glucose oxidase as the enzyme label, the colorimetric indicator composition can include a coupler and oxidizable compound which react to provide a dye. Alternatively, the composition can include a leuco dye and peroxidase or another suitable peroxidative compound which generates a detectable dye as a result of the formation of hydrogen peroxide produced when glucose oxidase converts glucose to gluconic acid. Useful leuco dyes are known in the art and include those, for example, described in U.S. Patents 4,089,747 and 4,670,385. The particular amounts of the colorimetric indicator composition and its various components are within the skill of a worker in the art.

The layers of the element can contain a variety of other desirable but optional components, including surfactants, thickeners, buffers, hardeners, antioxidants, coupler solvents, and other materials known in the art. The amounts of these components are also within the skill of a worker in the art.

The immunoassay can be manual or automated. In general, the amount of analyte in a liquid sample is determined by taking the element from a supply roll, chip packet or other source and physically contacting a finite area of the spreading layer with a sample of the liquid, for example 1 to 100 ml. The finite area which is contacted is generally no more than 100 mm$^2$.

After sample application in either embodiment, the element is exposed to any conditioning, such as incubation, heating or the like, that may be desirable to quicken or otherwise facilitate obtaining the test result.

The amount of T4 is determined by passing the element through a suitable apparatus for detecting the complexed ligand analogue directly or the detectable species formed as a result of enzymatic reaction of an enzyme label and a substrate. For example, the species can be detected with suitable radiometric, fluorometric or spectrophotometric apparatus using generally known procedures. In an enzymatic reaction, the resulting product is determined by measuring, for example, the reflection or transmission density or fluorescence in the center of the finite area which was contacted with the test sample. The area which is measured is generally from 3 to 5 mm in diameter for competing assays. The amount of T4 in the liquid sample is inversely proportional to the amount of label measured in the center of the finite area. As mentioned hereinbefore, in a preferred embodiment a separate wash step is required in order to separate complexed T4 from uncomplexed T4 (radial wash). Generally, label measurement is carried out after from 5 to 180 seconds after sample contact and spreading or application of the wash liquid.

The following example using ALP labeled T4 will serve to clarify the broad applicability of the present invention to any label for T4. The embodiment of the method used in the example to purify the immunoreactive components of a T4-ALP preparation uses a T3 affinity column. However other antibodies raised against T4 analogues as specified herein can also be used. T3 antibodies were immobilized on agarose beads according to standard protocols and a chromatography column was prepared. See Affinity Chromatography: A Practical Approach, Dean and others IRL Press Oxford England 1985.A buffer solution, pH 4 to 9, containing the T4-ALP (0.001 to 10 mg/mL) preparation was applied to the column. Antibodies are often adversely affected at pH level above or below 4 to 9. All non-immunoreactive components of the T4-ALP preparation passed directly through the column. The components retained by the column were immunoreactive. These components were removed from the column by passing a buffer containing T4 over the column. The T4 was present at a concentration of $10^{-5}$M to $10^{-3}$M. The T4 in solution competes with the T4-ALP for the binding sites of the T3 antibody. Because there was excess T4 in the buffer solution, the T4-ALP was displaced and eluted off the column. Excess T4 in the immunoreactive T4-ALP that was collected from the column was removed by dialysis.

EXAMPLE

Preparation of T4-ALP Conjugate

T4-ALP was prepared by reacting thiolated alkaline phosphatase with T4 that had been activated with the heterobifunctional agent SMCC (succinimidyl 4-(N-maleimidomethyl)cyclohexane-1-carboxylate). This procedure is described in Clinical Chemistry 30, Ito and others, pages 1682-1685 (1984). Other useful methods of making labeled conjugates are well known. See for example Practice and Theory of Enzyme Immunoassays: Laboratory Techniques in Biochemistry and Molecular Biology, Tijssen, Elsevier Science Publishers, Amsterdam, The Netherlands, 1985.

Immunoreactivity Test

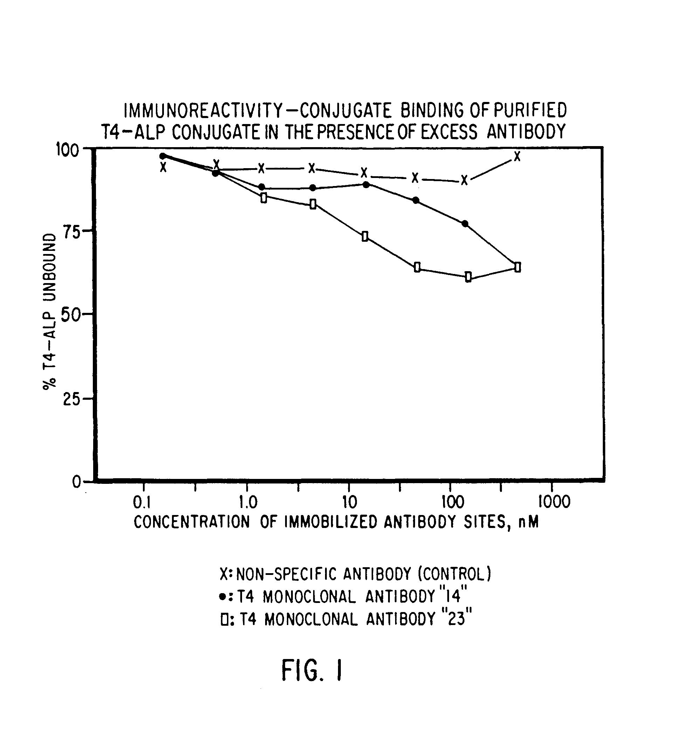

Mouse monoclonal T4 antibodies (lots 14 and 23) were covalently immobilized on one micron polymeric beads. A solution (in buffer) of the T4-ALP conjugate was prepared and aliquoted into a series of test tubes. Increasing amounts of the T4 antibody immobilized on beads were added to a series of tubes and incubated with the T4-ALP solutions for one hour at room temperature. The solutions were centrifuged to remove the immobilized antibody and any T4-ALP bound to the immobilized T4 antibodies. Samples of the supernatant were removed and assayed for alkaline phosphatase activity by adding a buffer solution (2-amino-2-methyl-1-propanol, pH 10) containing the alkaline phosphatase substrate p-nitrophenyl phosphate. Enzyme activity was monitored by observing the color change at 405 nm. Solutions of known alkaline phosphatase activity were used as standards to predict the concentrations of ALP in the supernatants. Plots of the amount of ALP in the supernatants versus the amount of added T4 antibody were prepared to determine the percent of immunoreactive T4-ALP conjugate in the preparations. Typically, the unpurified T4-ALP preparations contained about 70% non-immunoreactive material (30% immunoreactive material, see Figure 1). Control experiments using immobilized non-specific mouse monoclonal antibody on beads were used to check for non-specific binding of the T4-ALP preparation to the beads. No non-specific binding was observed (Figure 1) in these experiments.

Preparation of Immobilized T3 Antibodies for Affinity Chromatography

One gram of CNBr-activated-agarose beads (Sigma Chemical Company) was swollen in 15 mls of 0.001 M HCl for 30 minutes at room temperature. The swollen beads were washed with 200 mls 0.001 M HCl and then washed with 0.1 M sodium bicarbonate, 0.5 M NaCl, pH 7.9. The beads were then suspended in 5 mls of 0.1 M sodium bicarbonate, 0.5 M NaCl, pH 7.9. Three and four tenths (3.4) milligrams of a mouse monoclonal T3 antibody (Cambridge, Lot A1641) was added to the suspended beads and mixed overnight at room temperature. The beads were then washed with 100 mls of 0.1 M sodium bicarbonate, 0.5 M NaCl, pH 7.9. The beads were next washed with a 100 mls of 1 M ethanolamine (pH 8.0) and then incubated with 15 mls of 1M ethanolamine (pH 8.0) for 2 hours at room temperature. The beads were then washed with 3 cycles of first 50 mls of 0.1 M sodium acetate, 1.0 M NaCl, pH 4.0, and then 50 mls of 0.1 M borate, 1.0 M NaCl, pH 8.0. The agarose immobilized T3 antibodies were finally washed with 100 mls of 0.01 M phosphate, 0.15 M NaCl, pH 7.0.

The agarose beads with immobilized T3 antibodies were poured into a glass column (1 cm in diameter) to a height of 1 cm. The column was then washed extensively with 0.01M phosphate buffered saline (PBS, 0.01 M phosphate, 0.15 M NaCl, pH 7.4).

Affinity Purification of T4-ALP

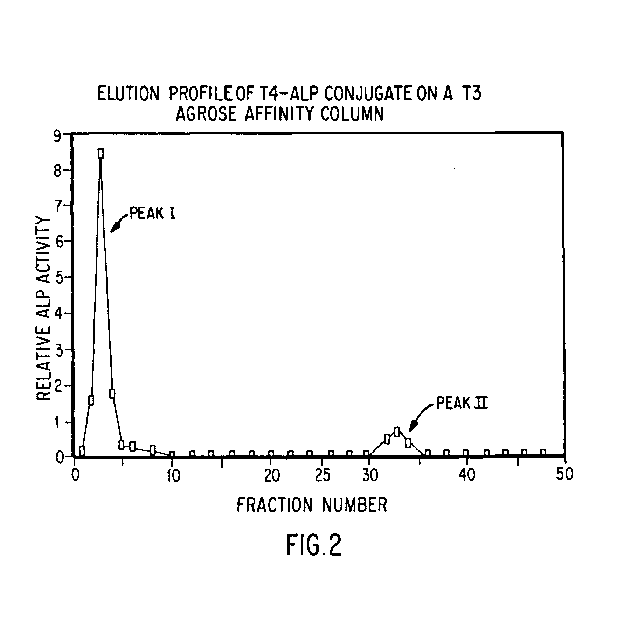

One ml of a solution of T4-ALP (2.4 mg/ml) in PBS was applied to the affinity column. The column was then first eluted with PBS. After 25 mls of PBS had passed through the column, the eluent was changed to PBS containing 0.0001 M T4. Thirty-five mls of this buffer was eluted. One ml fractions were collected throughout the entire elution and subsequently each fraction was analyzed for alkaline phosphatase activity. The results of these assays are plotted in Figure 2. Two peaks of alkaline phosphatase activity were recovered and designated Peak I (the activity passed by the column) and Peak II (the activity retained by the column and eluted after the addition of T4 to the buffer).

Immunoreactivity of Peak I and Peak II Elutions

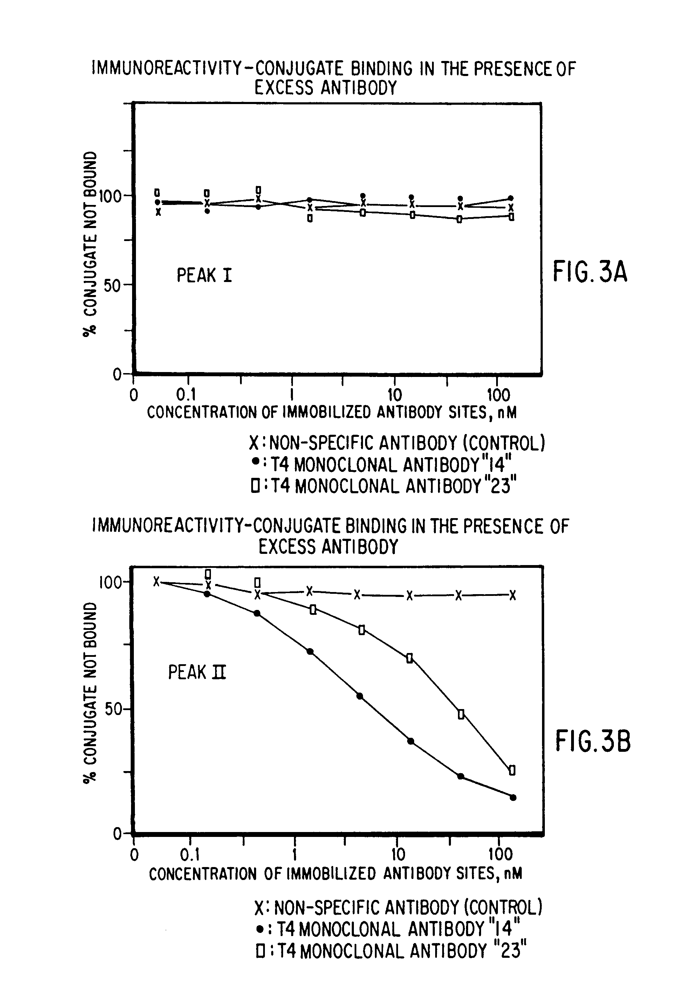

Samples of Peak I and Peak II elutions were tested for immunoreactivity as described above. Figure 3a shows that Peak I elutions were not immunoreactive. That is, there was no alkaline phosphatase activity removed even at the highest concentration of immobilized T4 antibody added. This result was as expected since Peak I was not retained by the affinity column. Figure 3b shows that Peak II elutions were about 90% immunoreactive. This confirms that the immunoreactive components of the T4-ALP preparation were retained by the column and eluted by the addition of T4 to the eluent. Also 100% immunoreactivity can be achieved by adding more immobilized antibodies to the reaction mixture.

Increased Signal in T4 Immunoassay Using Affinity Purified T4-ALP

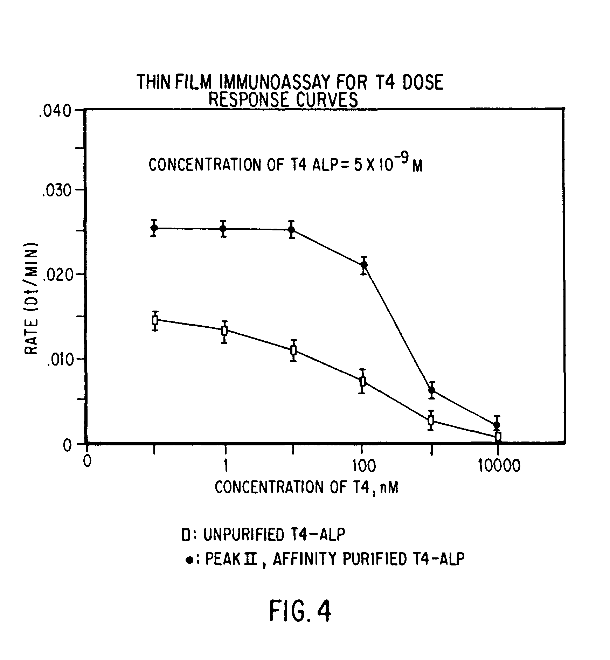

The purified T4-ALP was tested in an immunoassay for T4 that is based on a thin film format of the type described supra. The spreading layer of the thin film contains T4 antibodies immobilized on polymeric beads. Ten microliters of a test sample containing T4 and T4-ALP is applied to the spreading layer. The film is then incubated for five minutes at 37°C during which the competition reaction between the T4 and the T4-ALP and the immobilized antibody occurs. After this five minute incubation, ten microliters of a wash solution is applied which causes any T4-ALP that is not bound to immobilized antibody to be washed from the center of the spot. This second fluid also contains substrate for ALP (p-nitrophenyl phosphate) so that the activity of the enzyme which is retained in the center of the spot by immobilized antibody can be quantitated by measuring the color change using 400 nm light. This measurement is done at 37°C using reflectance densitometry. As in the previously described immunoassays, the amount of enzyme bound to the immobilized antibody is inversely correlated with the amount of T4 in the sample. Thus, solutions with low T4 concentrations will have higher measured color changes when applied to these films than solutions with high T4 concentrations. This is observed in the data in Figure 4. Figure 4 also compares the signals that are observed when either unpurified or affinity purified T4-ALP is added to the samples before they are applied to the thin film. It can be seen that higher signals are obtained using affinity purified T4-ALP than unpurified T4-ALP when the same concentration of alkaline phosphatase activity is added to the samples.

**Claims**

1. A method for purifying immunoreactive labeled thyroxine conjugates comprising the steps of:
   (a) immobilizing antibodies that have an affinity for thyroxide (T4);
   (b) preparing a crude liquid mixture of labeled thyroxine conjugate;
   (c) passing the liquid mixture of labeled thyroxine conjugate over the immobilized antibodies;
   (d) washing the immobilized antibodies with a buffer solution having a pH of 4 to 9; and
   (e) passing a buffered solution of thyroxine, having a pH of 4 to 9 over the immobilized antibodies, thereby removing immunoreactive labeled thyroxine conjugates characterized in that the immobilized antibodies are i) raised against an analogue of thyroxine (T4) and ii) cross-reactive with thyroxine.

2. A method according to claim 1 wherein the antibodies are raised against a thyroxine analogue selected from 3,3',5-triiodothyronine (T3); 3,5-diiodothyronine (T2); or 3,3',5'-triiodothyronine.

3. The method of claim 2 wherein the antibodies are raised against T3.

4. A method according to any one of the preceding claims wherein the antibodies are immobilized in a column on beads selected from polymeric beads, agarose beads, cellulose beads, dextran beads or glass beads.

5. The method of claim 4, wherein the antibodies are immobilized on agarose beads.

6. The method of Claim 1, 2, or 3, wherein the label is an enzyme.

7. The method of Claim 6, wherein the label is alkaline phosphatase or horseradish peroxidase.

8. The method of Claim 1, 2, or 3 wherein the concentration thyroxine in the buffered solution is $10^{-5}$ to $10^{-3}$ M and the pH of the buffer solution is 4 to 9.

9. A labeled thyroxine conjugate preparation wherein the conjugate is greater than 75% immunoreactive.

10. The preparation of Claim 9, wherein the conjugate is between 75 and 100% immunoreactive.

11. An immunoassay in which a labeled T4 conjugate according to Claim 9 or 10 is used.

12. The method of Claim 11, wherein the label is an enzyme.

13. The method of Claim 11, wherein the label is alkaline phosphatase or horseradish peroxidase.

14. A method for purifying immunoreactive labeled thyroxine conjugates comprising the steps of:
    (a) immobilizing antibodies having an affinity for thyroxide (T4) in a column on agarose beads;
    (b) preparing a crude liquid mixture of labeled thyroxine conjugate;
    (c) passing the liquid mixture of labeled thyroxine conjugate over the immobilized antibodies;
    (d) washing the immobilized antibodies with a buffer solution having a pH of 4 to 9; and
    (e) passing a buffered solution of thyroxine over the immobilized antibodies, thereby removing immunoreactive labeled thyroxine conjugates characterized in that the antibodies are raised against triiodothyromine.

15. A dry multilayer analytical element for conducting immunoassays comprising a labeled T4 conjugates according to claim 9 or 10.

IMMUNOREACTIVITY—CONJUGATE BINDING OF PURIFIED
T4—ALP CONJUGATE IN THE PRESENCE OF EXCESS ANTIBODY

X: NON—SPECIFIC ANTIBODY (CONTROL)
●: T4 MONOCLONAL ANTIBODY "14"
□: T4 MONOCLONAL ANTIBODY "23"

FIG. I

ELUTION PROFILE OF T4–ALP CONJUGATE ON A T3
AGROSE AFFINITY COLUMN

FIG.2

IMMUNOREACTIVITY–CONJUGATE BINDING IN THE PRESENCE OF
EXCESS ANTIBODY

PEAK I

FIG.3A

X : NON-SPECIFIC ANTIBODY (CONTROL)
● : T4 MONOCLONAL ANTIBODY "14"
□ : T4 MONOCLONAL ANTIBODY "23"

IMMUNOREACTIVITY–CONJUGATE BINDING IN THE PRESENCE OF
EXCESS ANTIBODY

PEAK II

FIG.3B

X : NON-SPECIFIC ANTIBODY (CONTROL)
● : T4 MONOCLONAL ANTIBODY "14"
□ : T4 MONOCLONAL ANTIBODY "23"

THIN FILM IMMUNOASSAY FOR T4 DOSE
RESPONSE CURVES

CONCENTRATION OF T4 ALP = $5 \times 10^{-9}$ M

□: UNPURIFIED T4-ALP
●: PEAK II, AFFINITY PURIFIED T4-ALP

FIG. 4

**European Patent Office**

**EUROPEAN SEARCH REPORT**

Application Number

**EP 91 20 1830**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 021 050 (BOEHRINGER MANNHEIM, GmbH) − − − | | G 01 N 33/535<br>G 01 N 33/78<br>G 01 N 33/546<br>G 01 N 33/548<br>G 01 N 33/58 |
| D,A | EP-A-0 026 103 (THE RADIOCHEMICAL CENTRE LTD) − − − − − | | |

|  |  |
|---|---|
| | TECHNICAL FIELDS SEARCHED (Int. Cl.5) |
| | G 01 N |

The present search report has been drawn up for all claims

| Place of search | Date of completion of search | Examiner |
|---|---|---|
| The Hague | 16 October 91 | CARTAGENA Y ABELLA P |